# EUROPEAN PATENT SPECIFICATION

(11) **EP 1 739 425 B1**
(45) Date of publication and mention of the grant of the patent: **09.09.2009**
(21) Application number: 05728425.9
(22) Date of filing: 30.03.2005
(51) Int. Cl.: G01N 33/50, G01N 33/15, G01N 33/68

(54) **ASSAY METHOD OF IDENTIFYING DRUG CANDIDATE**
TESTVERFAHREN ZUR IDENTIFIZIERUNG EINES KANDIDATENARZNEISTOFFS
TECHNIQUE D'ESSAI POUR IDENTIFIER UN CANDIDAT MEDICAMENT

(30) Priority: 31.03.2004 JP 2004107746
(43) Date of publication of application: 03.01.2007
(73) Proprietor: AnGes MG, Inc., Ibaraki-shi Osaka 567-0085 (JP)
(72) Inventor: SATO, Naoyuki, Ibaraki-shi, Osaka 567-0085 (JP); OKOCHI, Masayasu, Ibaraki-shi, Osaka 567-0085 (JP); TANIYAMA, Yoshiaki, Ibaraki-shi, Osaka 567-0085 (JP); OGIHARA, Toshio, Ibaraki-shi, Osaka 567-0085 (JP); MORISHITA, Ryuichi, Ibaraki-shi, Osaka 567-0085 (JP)
(74) Representative: Wichmann, Hendrik
(86) International application number: PCT/JP2005/006728
(87) International publication number: WO 2005/095958

(56) References cited:
- WO-A2-02/059150
- JP-A- 2002 522 747
- JP-A- 2002 536 963
- JP-A- 2003 199 760
- JP-A- 2003 325 516
- US-A1- 2003 027 210
- US-A1- 2003 083 277
- CHERNY R A ET AL: "AQUEOUS DISSOLUTION OF ALZHEIMER'S DISEASE ABETA AMYLOID DEPOSITS BY BIOMETAL DEPLETION" JOURNAL OF BIOLOGICAL CHEMISTRY, AMERICAN SOCIETY OF BIOLOCHEMICAL BIOLOGISTS, BIRMINGHAM,, US, vol. 274, no. 33, 1999, pages 23223-23228, XP000929630 ISSN: 0021-9258
- CALLAHAM M A ET AL: "REVERSIBILITY OF SCRAPIE-ASSOCIATED PRION PROTEIN AGGREGATION" JOURNAL OF BIOLOGICAL CHEMISTRY, AMERICAN SOCIETY OF BIOLOCHEMICAL BIOLOGISTS, BIRMINGHAM,, US, vol. 276, no. 30, 27 July 2001 (2001-07-27), pages 28022-28028, XP001094078 ISSN: 0021-9258
- ONO K ET AL: "Curcumin has potent anti-amyloidogenic effects for Alzheimer's beta-amyloid fibrils in vitro" JOURNAL OF NEUROSCIENCE RESEARCH, WILEY-LISS, US, vol. 75, no. 6, 15 March 2004 (2004-03-15), pages 742-750, XP002989615 ISSN: 0360-4012
- SATO ET AL: "Development of new screening system for Alzheimer disease, in vitro Abeta sink assay, to identify the dissociation of soluble Abeta from fibrils" NEUROBIOLOGY OF DISEASE, BLACKWELL SCIENCE, OXFORD, GB, vol. 22, no. 3, June 2006 (2006-06), pages 487-495, XP005485401 ISSN: 0969-9961
- TERADA H.: 'SONICATOR (Choonpashoriki)' KAGAKU TO SEIBUTSU, JIKKEN LINE 48, TANPAKUSHITSU TO KAKUSAN NO BUNRI SEISEI 15 June 2001, page 20, XP002997618
- ONO K. ET AL: 'Potent Anti-Amyloidogenic and Fibril-Destabilizing Effects of Polyphenols in Vitro: Implications for the Prevention and Therapeutics of Alzheimer's Disease' J. NEUROCHEM. vol. 87, no. 1, 2003, pages 172 - 181, XP002989614
- ONO K. ET AL: 'Curcumin has potent anti-amyliodogenic effects for Alzheimer's beta-amyloid fibrils in Vitro' J. NEUROSCI. RES. vol. 75, no. 6, 15 March 2004, pages 742 - 750, XP002989615

## Description

### Technical Field

The present invention relates to an in vitro method of identifying a drug candidate capable of removing peptide, oligopeptide, polypeptide or protein from fibril or aggregate, as defined in the claims.

### Background Art

Senile plaque containing amyloid β (hereinafter to be also referred to as Aβ) is one of the pathological features of Alzheimer's disease.

Reduction of Aβ is considered to be a primary therapeutic target.

There are reports on amyloid clearance after immunization with anti-Aβ antibodies.

Recent data reveal that the antibodies may act as peripheral sink for Aβ to alter the periphery/brain dynamics.

The factual disappearance of senile plaque indicates that the Aβ aggregation process is reversible.

However, there are no evidence of dissolution of soluble Aβ from aggregated Aβ.

Here, the present inventors have developed a sink-like Aβ assay for the analysis of *in vitro* transfer (replacement) of fibril to the supernatant following centrifugation, by which not only dissolution of soluble Aβ from aggregated Aβ but also promotion of the dissolution of Aβ by ultrasonication are indicated.

A compound or ultrasonication that promotes dissolution of Aβ in the *in vitro* sink-like Aβ assay may become a potential alternative to the treatment of Alzheimer's disease.

Alzheimer's disease (AD) is a neurodegenerative disease characterized by the presence of senile plaques in brain.

Aβ peptide consisting of 40-42 amino acids is the main component of senile plaque.

The inheritable character of this disease is related to the mutation of amyloid precursor protein (APP) and presenilin gene.

The mutation in these genes relating to the disease increases production of Aβ (1-42) predominantly present in the senile plaques.

Recently, immunization of mutant APP transgenic mouse with Aβ has been shown to be effective for suppressing plaque deposition (Schenk, Barbour et al. 1999).

Both plaques and neuritic lesions were reversible after administration of a single dose of Aβ antibody (Lombardo, Stern et al. 2003).

In addition to the Aβ antibody, a peripheral treatment with a pharmaceutical agent having high affinity for Aβ (gelsolin or GM1) reduced the level of Aβ in the brain (Matsuoka, Saito et al. 2003).

It has been proposed that generation of a peripheral sink mechanism via administration of antibodies is useful for treating a number of disorders characterized by the abnormal protein accumulation in an extracellular space (DeMattos, Bales et al. 2001).

The Aβ aggregation process has been sufficiently studied *in vitro.*

However, the reverse phenomenon wherein Aβ is dissolved from aggregated Aβ has not been studied yet.

In this study, we developed a novel *in vitro* sink-like Aβ assay to study dissolution of Aβ from aggregated Aβ.

We found that soluble Aβ dissolved from aggregated Aβ.

In addition, ultrasonication promoted dissolution of Aβ.

The compound and/or ultrasonication that promoted dissolution of Aβ in the *in vitro* sink-like Aβ assay may become an alternative for the treatment of Alzheimer's disease.

WO94/10569 (JP-A-8-502587) discloses a method for diagnosing or monitoring a β-amyloid peptide (hereinafter to be also referred to as βAP)-related condition in a patient, said method comprising: measuring the amount of soluble βAP or βAP fragments in a patient sample; comparing the measured amount with a predetermined amount of βAP or βAP fragments; and assessing patient status based on the difference between the measured and predetermined βAP or βAP fragment amounts (claim 23).

WO00/26238 (JP-A-2002-531065) discloses a method of identifying an agent which is capable of preventing, reducing and/or reversing the conversion of prion protein to a β-form, the method comprising: providing a sample of a prion protein and comparing the amount of the β-form qualitatively or quantitatively in the presence and absence of a test agent (claim 50).

WO00/43791 (JP-A-2002-540383) discloses a method comprising: forming a solution containing a species capable of binding neurodegenerative disease aggregate-forming or fibril-forming species and one of a sample suspected of containing neurodegenerative disease aggregate-forming or fibril-forming species for a drug candidate for inhibition of neurodegenerative disease aggregate or fibril-formation; and, without transferring any components into the solution or removing the solution from its container, detecting aggregation in the solution characteristic of neurodegenerative diseases (claim 172).

For evaluation of a number of test compounds, however, these prior arts are not easy, not quick, not highly reliable or not economical methods.
Cherny et al. (J. Biol. Chem (1999), 274(33): 23223-23228) disclose the aqueous dissolution of Alzheimer's disease A beta amyloid deposits by biometal depletion.
US 2003/0083277 A1 relates to the use of insulin degrading enzyme for the treatment of Alzheimer's disease in patients. Callahan et al. (J. Biol. Chem. (2001), 276(30): 28022-28028) report the reversibility of Scrapie-associated prion protein aggregation.
However, in comparison with the method of the present invention, none of these documents neither mentions nor suggests using ultrasonication for achieving the equilibrium state, which accelerates the arrival of the equilibrium state.

### Disclosure of the Invention

The present inventors have conducted intensive studies in an attempt to establish a method of identifying a drug candidate usable for the treatment of a disease caused by aggregation of peptide, oligopeptide, polypeptide or protein. They have found that the Aβ aggregation process is reversible, and established a method capable of identifying a drug candidate capable of removing peptide, oligopeptide, polypeptide or protein from fibril or aggregate, by measuring, in the presence of a test compound, the concentration of a soluble peptide, a soluble oligopeptide, a soluble polypeptide or a soluble protein in an equilibrium state in a solvent.

Accordingly, the present invention provides the following.
(1) A method of identifying a drug candidate capable of removing peptide, oligopeptide, polypeptide or protein from fibril or aggregate, which comprises measuring, in the presence of a test compound, the concentration of a soluble peptide, a soluble oligopeptide, a soluble polypeptide or a soluble protein in an equilibrium state in a solvent, as defined in the claims.
(2) The method of the above-mentioned (1), wherein the disease is Alzheimer's disease (AD), or Parkinson's syndrome (PD) (2) The method of the above-mentioned (1), wherein the fibril or aggregate was formed *in vitro.*
(3) The method of any of the above-mentioned (1) to (2), wherein the ultrasonication is substantially unaccompanied by heat generation.
(4) The method of any of the above-mentioned (1) to (3), wherein the ultrasonication conditions include 5 repeats of a 30 second ultrasonication at 1 MHz, 2 W/cm², duty ratio 20% with a 10 second pause.
(5) A method of identifying a drug candidate capable of removing β-amyloid (Aβ) from fibril or aggregate formed *in vitro*, which comprises measuring, in the presence of a test compound, the concentration of soluble β-amyloid (Aβ) in an equilibrium state in a solvent, as defined in the claims.
(6) The method of the above-mentioned (5), wherein the fibril or aggregate consists of Aβ (1-40).
(7) The method of the above-mentioned (5), wherein the fibril or aggregate consists of Aβ (1-42).
(8) The method of any of the above-mentioned (5) to (7), wherein the equilibrium state is achieved under ultrasonication.
(9) The method of any of the above-mentioned (5) to (7), wherein the ultrasonication is substantially unaccompanied by heat generation.
(10) The method of any of the above-mentioned (5) to (9), wherein the ultrasonication conditions include 5 repeats of a 30 second ultrasonication at 1 MHz, 2 W/cm², duty ratio 20% with a 10 second pause.

### Brief Description of the Drawings

Fig. 1 shows experimental procedures of *in vitro* sink-like Aβ assay.
Fig. 2 shows dissolution of soluble Aβ from aggregated Aβ, as a result of sandwich ELISA for soluble Aβ (1-40) and Aβ (1-92).
Fig. 3 shows the results of mass analysis of dissolved Aβ (1-40) and Aβ (1-42).
Fig. 4 shows the results of Western blot analysis of dissolved Aβ (1-40).
Fig. 5 shows SEC analysis charts of dissolved Aβ (1-40), which show promoted dissolution of Aβ by ultrasonication.
Fig. 6 shows experimental procedures for ultrasonication.
Fig. 7 shows the results of sandwich ELISA of dissolved Aβ (1-40).
Fig. 8 shows SEC analysis charts of fibrillization and oligomerization of Aβ (1-42).

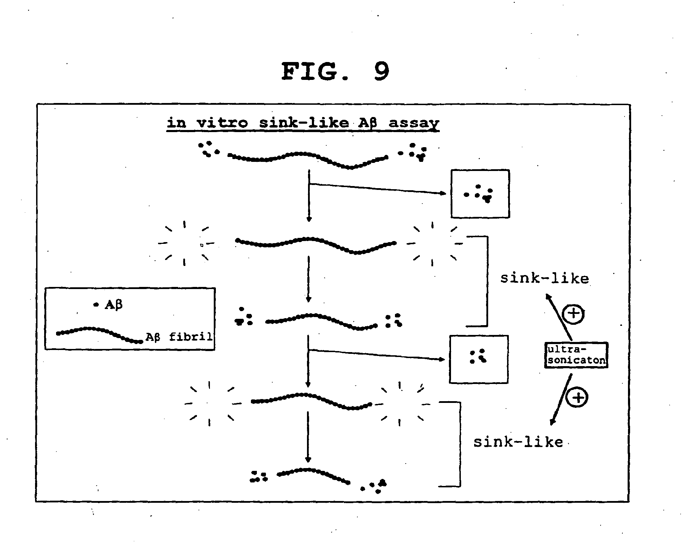

Fig. 9 is a schematic showing of the *in vitro* sink-like Aβ assay.
Fig. 10 shows the results of Example 2.

### Detailed Description of the Invention

The present invention is explained in detail in the following.

### <Identification method of drug candidates>

The present invention provides a method of identifying a drug candidate capable of removing peptide, oligopeptide, polypeptide or protein from fibril or aggregate, which comprises measuring, in the presence of a test compound (drug candidate), the concentration of a soluble peptide, a soluble oligopeptide, a soluble polypeptide or a soluble protein in an equilibrium state in a solvent, as defined in the claims.

In the step of the identification method of the present invention, the concentration of a soluble peptide, a soluble oligopeptide, a soluble polypeptide or a soluble protein in an equilibrium state in a solvent is measured in the presence of a test compound.

The test compound may be any known compound or novel compound and, for example, nucleic acid, carbohydrates, lipid, protein, peptide, organic low-molecular-weight compound, compound library prepared using combinatorial chemistry techniques, random peptide library prepared using solid phase synthesis or phage display method, natural components derived from microorganism, plants, animals, marine animals etc., and the like can be mentioned. Alternatively, the test compound may be one actually used for the treatment of a disease caused by aggregation of peptide, oligopeptide, polypeptide or protein.

The above-mentioned soluble peptide, oligopeptide, polypeptide and protein are not particularly limited as long as their aggregation can cause an adverse effect, and eventually a disease and, for example, Aβ, prion protein, polyglutamine, α synuclein, tau, superoxide dismutase-1 (SOD1), neuroserpin, amyloid precursor protein (APP) and the like can be mentioned.

Aβ is a main component constituting senile plaque, which is one of the major neuropathological changes in Alzheimer's disease, and produced by cleavage of amyloid precursor protein (APP) with two kinds of (β- and γ-) secretases. The major molecule species includes Aβ (1-40)(SEQ ID NO: 1) consisting of 40 amino acid residues and Aβ (1-42)(SEQ ID NO: 2) longer by 2 residues in the C-terminal side.

The aggregation or accumulation of Aβ is considered to cause diseases such as Alzheimer's disease, Down's syndrome and the like, and the accumulation sites thereof are senile plaque and cerebrovascular amyloid. Aggregation or accumulation of prion protein is considered to cause diseases such as Creutzfeldt-Jakob disease, Gerstmann-Sträussler syndrome, mad cow disease and the like, and the accumulation sites thereof are synapse, nerve cell and Kuru plaque amyloid. The aggregation or accumulation of polyglutamine is considered to cause diseases such as spinobulbar muscular atrophy, spinocerebellar ataxia (SCA), dentatorubral-pallidoluysian atrophy (DRPLA), Huntington chorea and the like, and the accumulation sites thereof are neuronal intranuclear inclusion bodies. The aggregation or accumulation of α-synuclein is considered to cause diseases such as Parkinson's syndrome, Lewy body dementia, multiple system atrophy and the like, and the accumulation sites thereof are Lewy body (intraneuronal) and oligodendrocyte (GCI). The aggregation or accumulation of tau is considered to cause diseases such as Alzheimer's disease, FTDP-17, progressive supranuclear palsy, corticobasal degeneration, Pick disease and the like, and the accumulation sites thereof are neurofibrillary tangle, glial inclusion bodies and Pick body. The aggregation or accumulation of superoxide dismutase-1 is considered to cause diseases such as familial amyotropic lateral sclerosis retinitis and the like, and the accumulation sites thereof are Lewy body-like inclusion bodies. The aggregation or accumulation of neuroserpin is considered to cause diseases such as familial dementia accompanying neuroserpin inclusion bodies and the like, and the accumulation sites thereof are Collins body (intraneronal). The aggregation or accumulation of ABri peptide is considered to cause diseases such as familial British dementia and the like, and the accumulation site thereof is cerebrovascular amyloid.

In the present invention, being in an "equilibrium state" means that the rate of dissolution of soluble peptide, soluble oligopeptide, soluble polypeptide or soluble protein from insoluble fibril or aggregate of peptide, oligopeptide, polypeptide or protein into a solvent is the same as the rate of aggregation of soluble peptide, oligopeptide, polypeptide or protein in the solvent to form insoluble fibril or aggregate, and they are balanced.

The step of the identification method of the present invention may include simply measuring, in the presence of a test compound, the concentration of soluble peptide, soluble oligopeptide, soluble polypeptide or soluble protein dissolved from fibril or aggregate in a solvent as defined in the claims.

In the present invention, by the fibril is meant a structure of about 1 µm in diameter where peptide and the like are linearly linked, and the aggregate means an assemblage of plural fibrils. The fibril and aggregate may be formed *in vitro* or *in vivo*. In consideration of the efficiency, operability, reproducibility of the test and the like of identification of a drug candidate, however, the fibril and aggregate formed *in vitro* is preferable.

In the present invention, by "being removed" means removal of soluble peptide, oligopeptide, polypeptide or protein from fibril or aggregate of aggregated peptide, oligopeptide, polypeptide or protein by dissolution.

For preparation of a sample in an equilibrium state, for example, soluble peptide, oligopeptide, polypeptide or protein, or fibril or aggregate thereof is added to a solvent and stood at an appropriate temperature (generally 0-40°C) until the equilibrium state is achieved. Arrival at the equilibrium state can be confirmed by measuring the concentration of soluble peptide, oligopeptide, polypeptide or protein in a solvent chronologically. When the equilibrium state is achieved, a test compound is added to the reaction system and the mixture is incubated at an appropriate temperature (generally about 37°C) (preferably for 1 day). After centrifugation at an appropriate temperature (generally 0-40°C) for 1-60 min, the obtained supernatant is subjected to an analysis.

For preparation of a sample in a non-equilibrium state, for example, peptide, oligopeptide, polypeptide or protein is added to a solvent, and the mixture is incubated at a suitable temperature (generally 0-40°C) for 1-6 hr to allow fibrillization or aggregation. When the fibrillization or aggregation is completed, the supernatant is separated by centrifugation at an appropriate temperature (generally 0-40°C) for 1-60 min and the like. Then, a test compound is added to the obtained supernatant, and the mixture is incubated at a suitable temperature (generally about 37°C) preferably for one day. After centrifugation at an appropriate temperature (generally 0-40°C) for 1-60 min, the supernatant is collected and subjected to an analysis.

The solvent to be used here for the method of the present invention is not particularly limited as long as it does not prevent the object of the present invention and, for example, buffers such as PBS, acetate buffer, citrate buffer, Tris-HCl buffer and the like are used. The solvent is preferably adjusted to about pH3-8, more preferably about pH6-8 from the aspect of physiological conditions.

The concentration of soluble peptide, oligopeptide, polypeptide or protein in a solvent can be measured by a method known *per* se. For example, it can be measured by methods such as ELISA, Western blot, mass analysis, size-exclusion chromatography (SEC) and the like.

When, as a result of the above-mentioned measurement, an increase in the concentration of soluble peptide, oligopeptide, polypeptide or protein in a solvent can be confirmed by the addition of the test compound, as compared to the absence of the test compound, the test compound can be determined to be a drug candidate capable of removing peptide, oligopeptide, polypeptide or protein from fibril or aggregate. The thus-obtained compound can be useful for the development of a drug for the prophylaxis or treatment of Alzheimer's disease (AD), or Down's syndrome (DS). A compound obtained by the identification method of the present invention is also disclosed.

According to the identification method, a drug candidate capable of specifically removing peptide, oligopeptide, polypeptide or protein from fibril or aggregate of particular peptide, oligopeptide, polypeptide or protein can be obtained by appropriately selecting the kind of peptide, oligopeptide, polypeptide or protein. Such drug candidate can be useful for the prophylaxis or treatment of a disease caused by the aggregation of the particular peptide, oligopeptide, polypeptide or protein.

### <Ultrasonication>

The above-mentioned equilibrium state may be achieved under ultrasonication. Preferably, the ultrasonication does not substantially accompany heat generation. While the ultrasonication is not particularly limited, generally, 15 repeats of a 10 second ultrasonication under the conditions of 1-20 MHz, 0.1-10 W/cm², duty ratio 10-90% with a 10 second pause are performed, more preferably, 5 repeats of a 30 second ultrasonication under the conditions of 2.5-7.5 MHz, 0.5-5 W/cm², duty ratio 20-70% with a 10 second pause are performed, wherein the duty ratio means transmission time/(transmission time + transmission interval).

The above-mentioned ultrasonication promotes removal of peptide, oligopeptide, polypeptide or protein from fibril or aggregate. Therefore, ultrasonication accelerates arrival at the equilibrium state, and enables more rapid identification of a drug candidate.

Moreover, a method of removing peptide, oligopeptide, polypeptide or protein from fibril or aggregate by ultrasonication is also disclosed.

Furthermore, there is also disclosed a method for the treatment of a disease caused by aggregation of peptide, oligopeptide, polypeptide or protein, which comprises application of ultrasonication to a patient.

An apparatus for treating a disease caused by the aggregation of peptide, oligopeptide, polypeptide or protein is disclosed, which comprises a means for ultrasonicating an affected part to remove peptide, oligopeptide, polypeptide or protein from fibril or aggregate.

For ultrasonication here, generally, 15 repeats of a 10 second ultrasonication under the conditions of 1-20 MHz, 0.1-10 W/cm², duty ratio 10-90% with a 10 second pause are performed, more preferably, 5 repeats of a 30 second ultrasonication under the conditions of 2.5-7.5 MHz, 0.5-5 W/cm², duty ratio 20-70% with a 10 second pause are performed.

### <Dissolution promoter>

A dissolution promoter for removing peptide, oligopeptide, polypeptide or protein from fibril or aggregate is disclosed, which contains, as an active ingredient, a compound obtained by the above-mentioned identification method.

As is clear from the following Examples, as a compound obtained by the above-mentioned identification method, ferric dehydroporphyrin IX, amphotericin B, myricetin, tannic acid, curcumin, azure B and basic blue 41 are used.

Accordingly, a dissolution promoter for removing peptide, oligopeptide, polypeptide or protein from fibril or aggregate is disclosed, which contains, as an active ingredient, at least one selected from ferric dehydroporphyrin IX, amphotericin B, myricetin, tannic acid, curcumin, azure B and basic blue 41.

Ferric dehydroporphyrin IX is a compound represented by the following chemical formula 1: 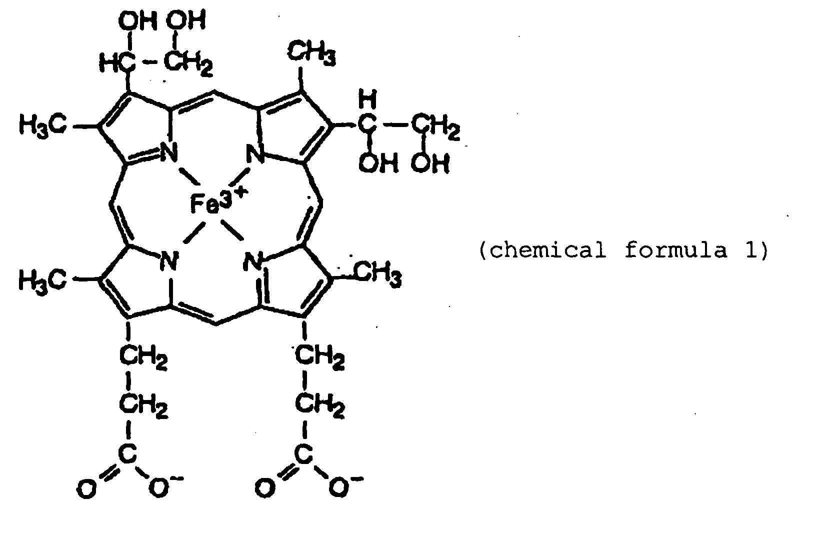 , which is reported to inhibit *in vitro* the formation of a protease-resistant protein (PrP-res) and to be possibly useful for the treatment of transmissible spongiform encephalopathy (S.A. Priola et al., Science, 287, 1503 (Feb 25, 2000)). There is also a report that the compound inhibits tau filament formation (S. Taniguchi et al., J Biol Chem, 280(9), 7614-7623, 2005).

Amphotericin B is a compound represented by the following chemical formula 2: 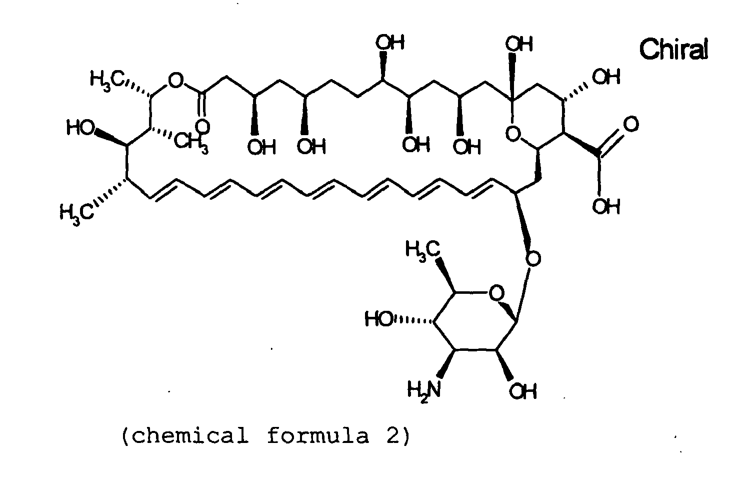 , which is known to delay Aβ fiber formation (S.C. Hartsel et al., Biochemistry 42, 6228 (May 27, 2003)).

Myricetin and tannic acid are each a compound represented by the following chemical formulas 3 and 4, respectively: 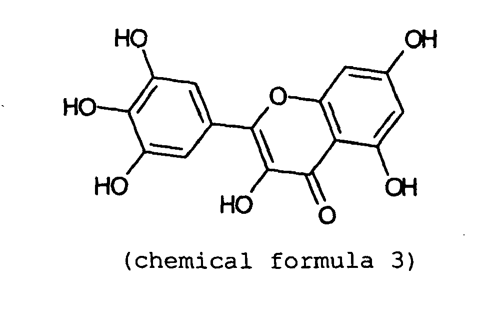 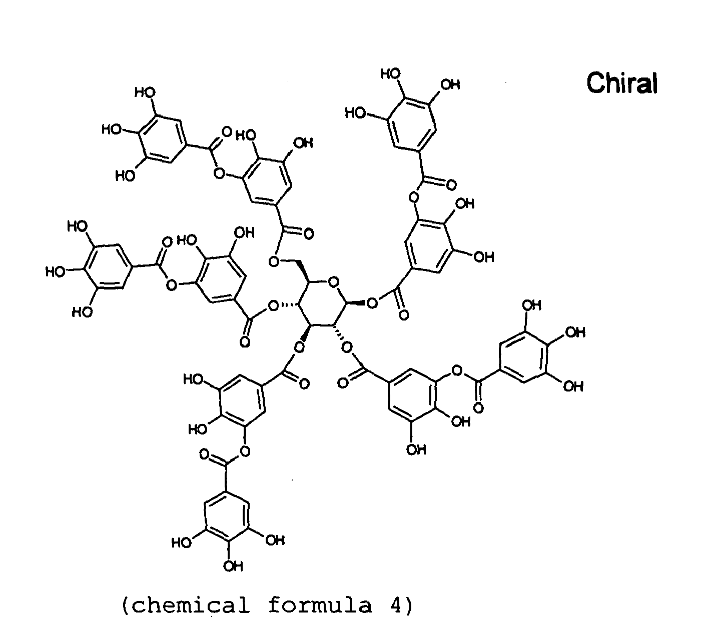 , which have been reported to show *in vitro* inhibitory action on the formation or elongation of Aβ (K. Ono, Biochim Biophys Acta 1690, 193 (Nov 5, 2004).

Curcumin is a compound represented by the following chemical formula 5: 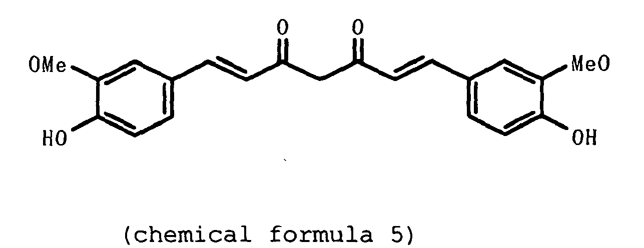 , which has been reported to inhibit the formation of an Aβ (1-40) fiber (F. Yang et al., J Biol Chem, 280(7), 5892-5901, 2005), and is known to be effective as a therapeutic drug for Alzheimer (G.P. Lim et al., J Neurosci, 21(21):8370-7 (Nov 1, 2001) and WO03/103583).

Azure B is a compound represented by the following chemical formula 6: 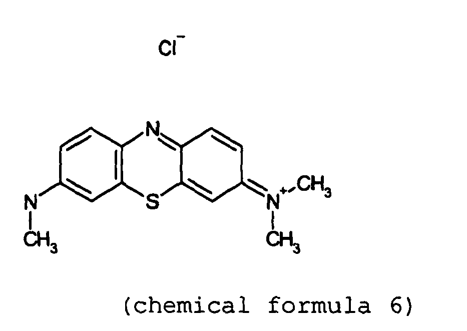 , which has been reported to inhibit tau filament formation (S. Taniguchi et al., J Biol Chem 280(9), 7614-7623, 2005).

Basic blue 41 is a compound represented by the following chemical formula 7: 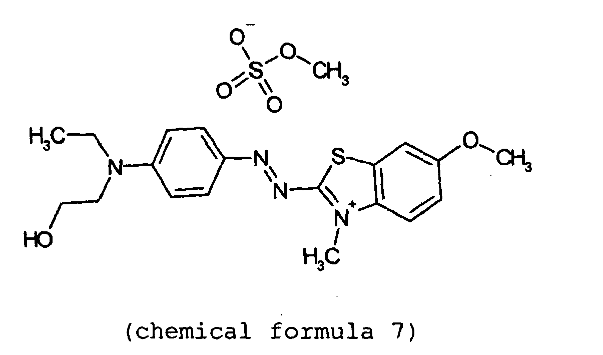 , which is one kind of thioflavine T, and thioflavine T is known to be a fluorescence indicator of amyloid fibers (H. Nakai et al, Lab Invest 62, 768 (Jun 1990)).

Since the dissolution promoter can enhance the action to remove peptide, oligopeptide, polypeptide or protein from fibril or aggregate, it is useful for the prophylaxis or treatment of a disease caused by the aggregation of peptide, oligopeptide, polypeptide or protein.

As described in the aforementioned publications, the above-mentioned compounds are all known to be usable for various diseases. Since the compounds were newly found to be able to dissolve peptide, oligopeptide, polypeptide or protein from fibril or aggregate in the present invention, it is suggested that dissolution promoters containing these compounds can be used for the prophylaxis or treatment of diseases caused by the aggregation of peptide, oligopeptide, polypeptide or protein.

The dissolution promoter can contain, in addition to the above-mentioned compound, optional carrier and the like. As the optional carrier, pharmaceutically acceptable carrier (mentioned later) can be mentioned.

The dissolution promoter can be used as a pharmaceutical agent or a test reagent and the like, or used *in vivo* or *in vitro.* When the dissolution promoter is used as a pharmaceutical agent, it can be orally or parenterally administered to animals (e.g., mammals such as human, simian, bovine, horse, dog, cat, rabbit, rat, mouse and the like), and can be used in the dosage form of oral administration agents such as tablet, capsule, troche, granule, powder and the like or in the dosage form of external preparations (liquid, lotion, suspension, emulsion, cream, ointment, gel etc.), injection, suppository and the like.

As a pharmaceutically acceptable carrier, for example, excipients such as sucrose, starch, mannit, sorbit, lactose, glucose, cellulose, talc, calcium phosphate, calcium carbonate and the like, binders such as cellulose, methylcellulose, hydroxypropylcellulose, polypropylpyrrolidone, gelatin, gum arabic, polyethylene glycol, sucrose, starch and the like, disintegrants such as starch, carboxymethylcellulose, hydroxypropyl starch, sodium glycol starch, sodium hydrogencarbonate, calcium phosphate, calcium citrate and the like, lubricants such as magnesium stearate, aerosil, talc, sodium lauryl sulfate and the like, aromatic such as citric acid, menthol, ammonium glycyrrhizinate, glycine, orange powder and the like, preservatives such as sodium benzoate, sodium bisulfite, methylparaben, propylparaben and the like, stabilizers such as citric acid, sodium citrate, acetic acid and the like, suspending agents such as methylcellulose, polyvinylpyrrolidone, aluminum stearate and the like, diluents such as surfactant and the like, dispersing agent, water, saline, orange juice and the like, base wax such as cacao butter, polyethylene glycol, white kerosene and the like, and the like can be mentioned, though not limited to these.

The preparation preferable for oral administration includes liquids obtained by dissolving an effective amount of a substance in a diluting solution such as water, saline and orange juice, capsule containing an effective amount of a substance as solid or granule, sachet or tablet, suspension wherein an effective amount of a substance is suspended in a suitable dispersion, an emulsion wherein a solution containing an effective amount of a substance is dispersed and emulsified in a suitable dispersion medium and the like.

As a preparation preferable for parenteral administration (e.g., subcutaneous injection, muscular injection, topical injection, intraperitoneal administration and the like), aqueous and non-aqueous isotonic sterile injections can be mentioned, which may contain antioxidant, buffer, antibacterial agent, isotonicity agent and the like. In addition, aqueous and non-aqueous sterile suspensions can be mentioned, which may include suspension, solubilizer, thickener, stabilizer, preservative and the like. The preparation can be sealed in a container by a unit dose or plural doses like ampoules and vials. In addition, an active ingredient and a pharmaceutically acceptable carrier may be lyophilized and preserved such that they only need to be dissolved or dispersed in a suitable sterile vehicle immediately before use.

While the dose of the dissolution promoter cannot be said in general, since it varies depending on the activity and kind of the active ingredient, severity of the disease, the animal species to be the subject of administration, and drug acceptability, body weight, age and the like of the subject of administration, it is generally about 0.001 - about 5 mg/kg/day for an adult based on the amount of the active ingredient.

### Examples

The present invention is explained in detail in the following by referring to Examples, which are not to be construed as limitative.

### Example 1

### (I) Method

### (1) Reagent and solvent

Unless otherwise specified, the drug was obtained from Sigma Ltd. or Wako.

Water was distilled twice, and deionized using a Milli-Q system (Millipore Corporation, Bedford, Massachusetts).

### (2) Peptide

Aβ (1-40) and Aβ (1-42) were obtained from PEPTIDE INSTITUTE, INC. (Osaka, Japan). Aβ solution was prepared anew as described in Hartley, Walsh et al. 1999, for every experiment. Aβ (1-40) peptide (0.55 mg) was dissolved in 1 mM NaOH (130 µl) containing 0.01% phenol red.

10 mM NaOH was added to adjust the solution to pH 7.5. To give a 500 µM Aβ solution, PBS/ddH20 was added. Undissolved Aβ was removed by centrifugation before separating the supernatant (15,000xg, 3 min).

### (3) Sample preparation

The 500 µM Aβ solution (20 µl) was taken in a 1.5 ml Eppendorf tube. The sample was incubated at 37°C for several time periods until completion of fibrillization. After centrifugation at 22°C (15,000xg, 10 min), the supernatant was removed.

The obtained pellets (i.e., Aβ fibril) were washed 3 times with phosphate buffer (PBS). Fresh PBS (20 µl) was carefully and gradually added to the pellets. The sample was incubated at 37°C for one day. After centrifugation at 22°C (15,000xg, 10 min), the supernatant (16 µl) was taken and subjected to the analysis. The remaining supernatant was removed. Fresh PBS (20 µl) was carefully and gradually added to the pellets.

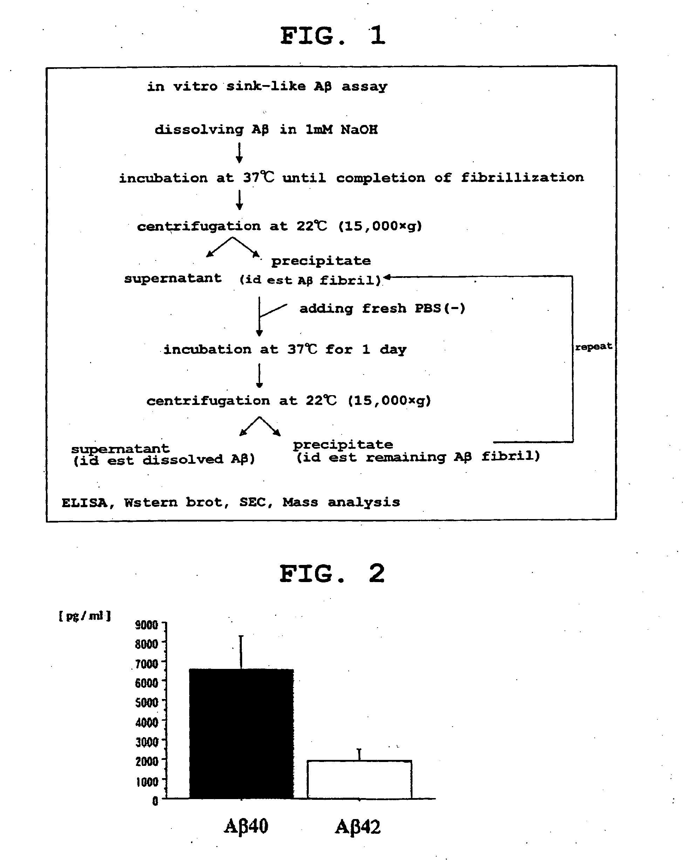

The above operation was repeated (see Fig. 1).

The sample was analyzed as in the following by ELISA, Western blot, mass analysis and size-exclusion chromatography (SEC).

### (4) Sandwich method Aβ ELISA

Sandwich ELISA for Aβ (1-40) and Aβ (1-42) was performed using an Aβ (1-40) ELISA kit and Aβ (1-42) ELISA kit (Biosoure, IBL, Signet Laboratories and the like).

### (5) SEC

Superose 6 column (Amersham) was set to the HPLC system consisting of an injector and UV detector. After elution from the column at 0.04 ml/min, peptide was detected by UV absorption at 218 nm.

Each experiment was performed at least twice. Prepacked column was washed with 2N NaOH between experiments.

For respective studies, an appropriate column was equilibrated with an elution buffer in an amount corresponding to at least 3-fold column volume, and then calibrated with 5 kinds of molecular weight standard product: chicken ovalbumin (44,000); horse myoglobulin (17,000); human γ-globulin (158,000); bovine siloglobulin (670,000); and vitamin B₁₂ (1,350).

As the elution buffer, 75 mM NaCl, 5 mM Tris-HCl (pH 7.4) were used.

### (6) Gel electrophoresis and Western blot

Using Tris/Tricine gel (Invitrogen), SDS polyacrylamide gel electrophoresis (SDS-PAGE) was performed. A sample was mixed with a 2xSDS sample buffer (Invitrogen), and rapidly boiled for 5 min prior to electrophoresis. The sample was transferred onto a polyvinylidene difluoride (PVDF) membrane (Millipore Corporation, Bedford, Massachusetts, US).

Western blot was performed using 6E10 antibody.

ECL was used as the detection system.

### (7) Mass analysis

Analysis of Aβ species by combined immunoprecipitation/matrix associated laser desorption ionization-time of flight (MALDI-TOF) mass spectrometry (IP/MS) was performed as described (Okochi, Steiner et al. 2002).

### (8) Ultrasonication

A sample tube was exposed to ultrasonication (2.5 W/cm²) at room temperature for 30 seconds (5 repeats of 30 sec ultrasonication).

### (II) Results

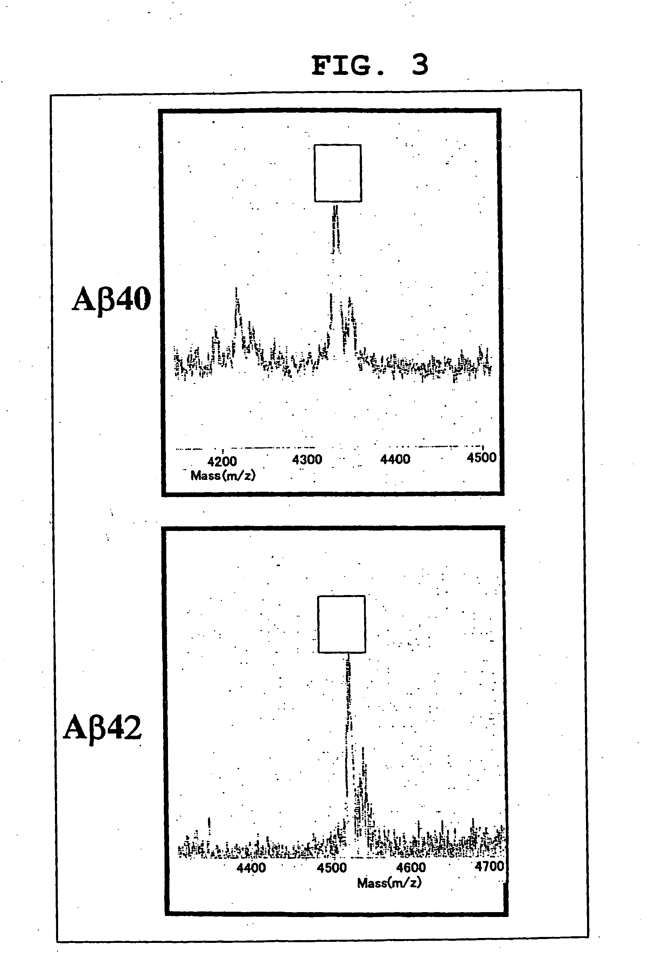

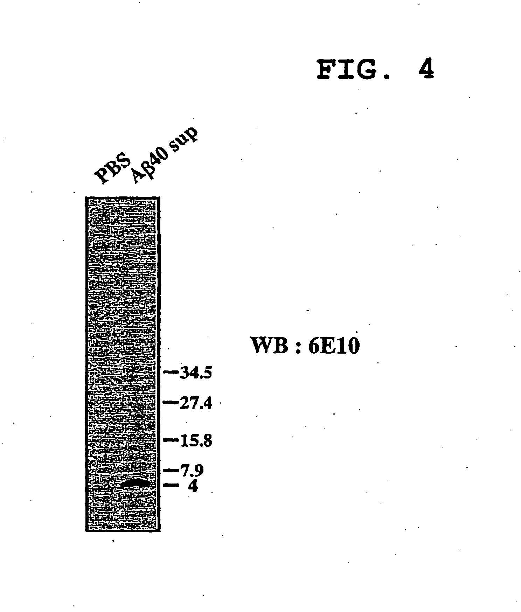

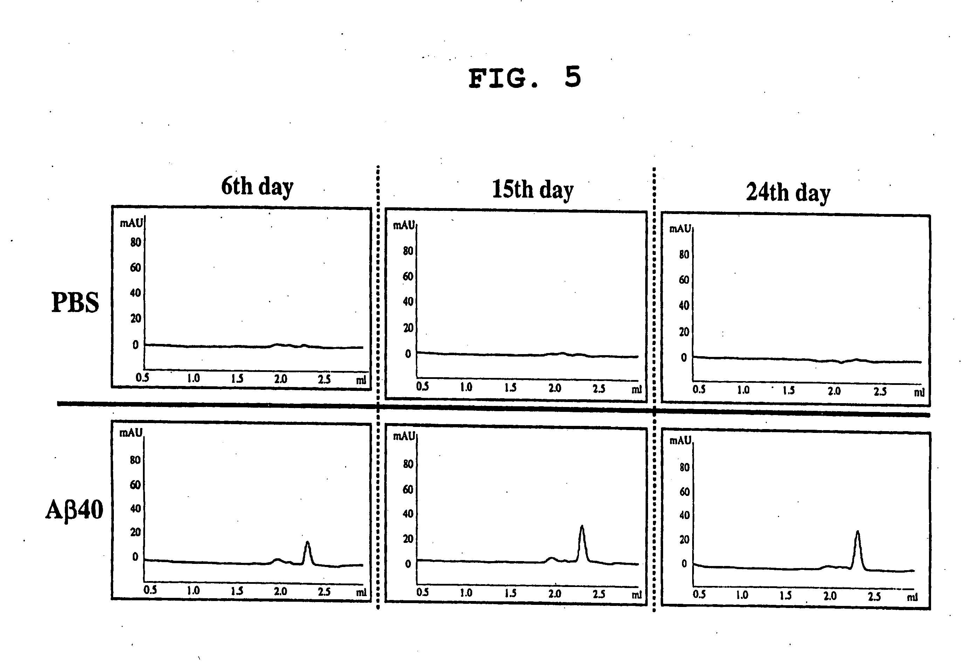

Soluble Aβ dissolved from aggregated Aβ.
(1) Sandwich ELISA for dissolved Aβ (1-40) and Aβ (1-42)
   Sandwich ELISA for dissolved Aβ (1-40) and Aβ (1-42) was performed according to the described experiment method. The amount of the dissolved Aβ (1-40) was 6562.5±1750.9 (S.E.) pg/ml (n=6). The amount of the dissolved Aβ (1-42) was 1939.7±607.6 (S.E.) pg/ml (n=6). The respective results are shown in Fig. 2.
(2) Mass analysis of dissolved Aβ (1-40) and Aβ (1-42)
   The molecular weight of dissolved Aβ (1-40) and Aβ (1-42) was confirmed to be 4329.8 and 4514.0, respectively, by mass analysis (Fig. 3).
(3) Western blot of dissolved Aβ (1-40)
   Detection of a nearly single band of low molecular weight is shown (Fig. 4).
(4) SEC analysis of dissolved Aβ (1-40) and Aβ (1-42)
   SEC analysis showed a sufficient single 2.4 ml peak (Fig. 5).
(5) Effect of ultrasonication
   Ultrasonication promoted Aβ dissolution.

A sample tube was exposed to ultrasonication (2.5 W/cm²) at room temperature, duty ratio 20% for 30 seconds (5 repeats of 30 sec ultrasonication).

Sandwich ELISA for dissolved Aβ (1-40) and Aβ (1-42) was performed.

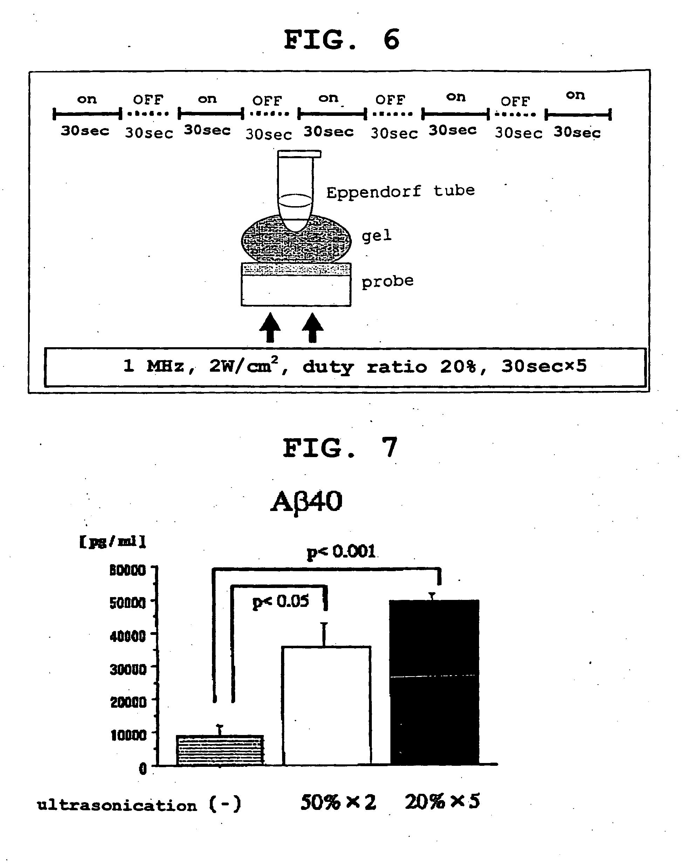

Ultrasonication for 30 seconds (5 repeats of 30 second ultrasonication at 2.5 W/cm², duty ratio 20%, room temperature) promoted dissolution of Aβ (1-40) (Fig. 6).
(control group: 8791.7±3121.1 (S.E.) pg/ml (n=3); ultrasonication group: 49479.3±2167.6 (S.E.) pg/ml (n=3); less than p 0.001)

In another experiment method at duty ratio 50% (30 seconds, 2.5 W/cm², room temperature, 2 repeats of 30 second ultrasonication), dissolution of Aβ (1-40) was promoted.
(control group: 8791.7±3121.1 (S.E.) pg/ml (n=3); ultrasonication group 50% × twice: 35520±7140 (S.E.) pg/ml (n=3); p less than 0.05)

The effect of ultrasonication is shown in Fig. 7. (6) SEC analysis of Aβ (1-42) fibrillization and oligomerization

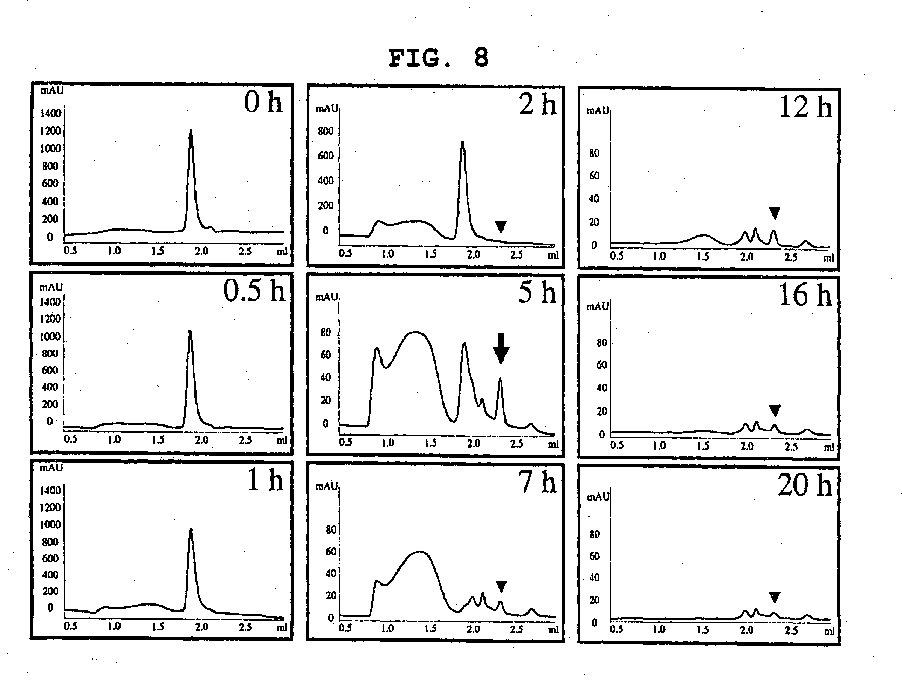

After dissolution, soluble Aβ (1-42) was immediately subjected to Superose 6 chromatography analysis by the experiment method as described. The results are shown in Fig. 8.

A peak derived from the gel (gel-included peak) was eluted at 1.9 ml.

The Aβ oligomer size in the peak could not be determined.

A large oligomer and a fibril precursor were observed during the time between 1 h and 12 h (arrow or triangle in the Figure).

The peak at 2.4 m increased at 5 h (arrow in the Figure).

Since this peak was observed when the dissolution of Aβ (1-40) was analyzed by SEC, it was eluted in the same fraction.

### Example 2

### (I) Method

### (1) Reagent and solvent

Ferric dehydroporphyrin IX, amphotericin B, myricetin, tannic acid, curcumin, azure B and basic blue 41 were each obtained from Sigma-Aldrich.

Except those mentioned above, the preparation followed Example 1.

### (2) Peptide

Peptide was prepared in the same manner as in Example 1, (I)-(2).

### (3) Sample preparation

A 500 µM Aβ (1-42) solution (20 µl) was separated in a 1.5 ml tube. The peptide was incubated for several days at 37°C until fibrillization was completed. After centrifugation at 22°C (15,000xg, 10 min), the supernatant was removed. The obtained pellets were gradually and carefully washed 3 times with PBS. Fresh PBS (20 µl) was carefully and gradually added to the pellets. The sample was incubated at 37°C for one day. After centrifugation (15,000xg, 10 min) at 22°C, the supernatant was removed. Then, fresh PBS (20 µl) containing a compound: ferric dehydroporphyrin IX, amphotericin B, myricetin, tannic acid, curcumin, azure B or basic blue 41, or fresh PBS (20 µl) without any compounds as a control were carefully and gradually added to the pellets. The sample was incubated at 37°C for one day. After centrifugation (15,000xg, 10 min) at 22°C, the supernatant (16 µl) (Aβ dissolved from fibril) was taken and subjected to Aβ ELISA analysis.

### (II) Results

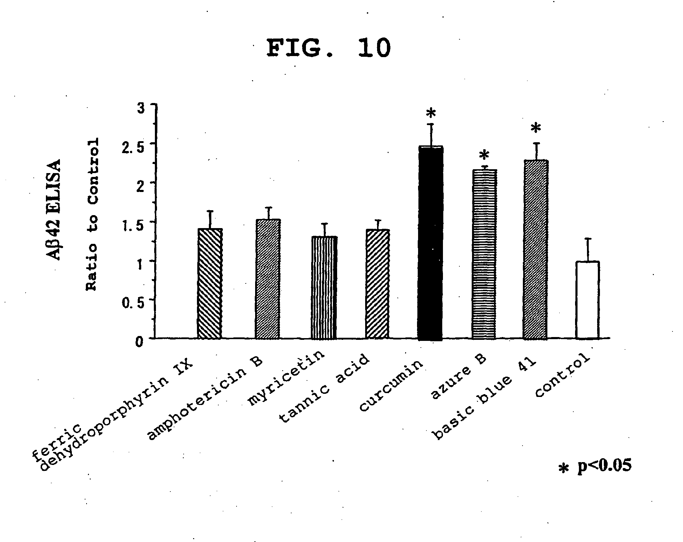

The results are shown in Table 1 and Fig. 10.

**Table 1**

| | average | SE |
|---|---|---|
| ferric dehydroporphyrin IX | 1.4124 | 0.224152 |
| amphotericin B | 1.5309 | 0.161446 |
| myricetin | 1.3196 | 0.16005 |
| tannic acid | 1.3969 | 0.123811 |
| curcumin | 2.4691 | 0.282381 |
| azure B | 2.1701 | 0.044954 |
| basic blue 41 | 2.299 | 0.220559 |
| control | 1 | 0.303872 |

The average value shows the ratio when the control is 1.

As shown in Table 1 and Fig. 10, when the above-mentioned compound was added, significant dissolution of Aβ was observed.

### References

1. DeMattos, R. B., K. R. Bales, et al. (2001). "Peripheral anti-A beta antibody alters CNS and plasma A beta clearance and decreases brain A beta burden in a mouse model of Alzheimer's disease." Proc Natl Acad Sci U S A 98(15): 8850-5.
2. Hartley, D. M., D. M. Walsh, et al. (1999). "Protofibrillar intermediates of amyloid beta-protein induce acute electrophysiological changes and progressive neurotoxicity in cortical neurons." J Neurosci 19(20): 8876-84.
3. Lombardo, J. A., E. A. Stern, et al. (2003). "Amyloid-beta antibody treatment leads to rapid normalization of plaque-induced neuritic alterations." J Neurosci 23(34): 10879-83.
4. Matsuoka, Y., M. Saito, et al. (2003). "Novel therapeutic approach for the treatment of Alzheimer's disease by peripheral administration of agents with an affinity to beta-amyloid." J Neurosci 23(1): 29-33.
5. Okochi, M., H. Steiner, et al. (2002). "Presenilins mediate a dual intramembranous gamma-secretase cleavage of Notch-1." Embo J 21(20): 5408-16.
6. Schenk, D., R. Barbour, et al. (1999). "Immunization with amyloid-beta attenuates Alzheimer-disease-like pathology in the PDAPP mouse." Nature 400(6740): 173-7.

### Industrial Applicability

As is clear from the foregoing explanation, the present invention as defined in the claims enables identification of a drug candidate capable of removing peptide, oligopeptide, polypeptide or protein from fibril or aggregate, by which the present invention is expected to be highly useful for the treatment, prophylaxis or study of the diseases caused by the aggregation of peptide, oligopeptide, polypeptide or protein, such as Alzheimer's disease, which have various repercussions in the modern society.

## Claims

1. An *in vitro* method of identifying whether a drug candidate is useful in the treatment of Alzheimer's disease (AD) or Down's syndrome comprising:
identifying whether said drug candidate is capable of removing peptide, oligopeptide, polypeptide or protein from fibril or aggregate by measuring, in the presence of said drug candidate, the concentration of the soluble peptide, soluble oligopeptide, soluble polypeptide or soluble protein dissolved from fibril or aggregate in a solvent;
wherein the soluble peptide, the soluble oligopeptide, the soluble polypeptide or the soluble protein is in an equilibrium state in the solvent;
wherein the equilibrium state is achieved when the rate of dissolution of soluble peptide, soluble oligopeptide, soluble polypeptide or soluble protein from insoluble fibril or aggregate of peptide, oligopeptide, polypeptide or protein into a solvent is the same as the rate of aggregation of soluble peptide, oligopeptide, polypeptide or protein in the solvent to form insoluble fibril or aggregate, and they are balanced; and
wherein the equilibrium state is achieved under ultrasonication.

2. The method of claim 1, wherein the fibril or aggregate was formed *in vitro.*

3. The method of claim 1 or 2, wherein the ultrasonication is substantially unaccompanied by heat generation.

4. The method of any of claims 1 to 3, wherein the ultrasonication conditions include 5 repeats of a 30 second ultrasonication at 1 MHz, 2 W/cm², duty ratio 20% with a 10 second pause.

5. The method of any of claims 1 to 4, wherein the peptide, oligopeptide, polypeptide or protein is β-amyloid (Aβ).

6. The method of any of claims 1 to 5, wherein the fibril or aggregate consists of Aβ (1-40) or Aβ (1-42).

## Patentansprüche

1. In-vitro-Verfahren zum Identifizieren, ob ein Wirkstoffkandidat in der Behandlung von der Alzheimer'schen Krankheit (Alzheimer's disease, AD) oder Down-Syndrom nützlich ist, umfassend:
Identifizieren, ob der Wirkstoffkandidat zum Entfernen von Peptid, Oligopeptid, Polypeptid oder Protein aus Fibril oder Aggregat fähig ist, durch Messen der Konzentration des löslichen Peptids, löslichen Oligopeptids, löslichen Polypeptids, oder löslichen Proteins, das aus dem Fibril oder Aggregat gelöst ist, in einem Lösungsmittel in der Gegenwart des Wirkstoffkandidaten;
Wobei das lösliche Peptid, das lösliche Oligopeptid, das lösliche Polypeptid oder das lösliche Protein in dem Lösungsmittel in einem Gleichgewichtszustand ist;
wobei der Gleichgewichtszustand erreicht wird, wenn die Auflösungsrate von löslichem Peptid, löslichem Oligopeptid, löslichem Polypeptid oder löslichem Protein aus unlöslichem Fibril oder Aggregat von Peptid, Oligopeptid, Polypeptid oder Protein in einem Lösungsmittel gleich ist wie die Aggregationsrate von löslichem Peptid, Oligopeptid, Polypeptid oder Protein in dem Lösungsmittel, um unlösliches Fibril oder Aggregat zu bilden, und sie ausgeglichen sind; und wobei der Gleichgewichtszustand unter Ultraschallbehandlung erreicht wird.

2. Verfahren nach Anspruch 1, wobei das Fibril oder Aggregat *in vitro* gebildet wurde.

3. Verfahren nach Anspruch 1 oder 2, wobei die Ultraschallbehandlung im Wesentlichen von Hitzebildung unbegleitet ist.

4. Verfahren nach einem beliebigen der Ansprüche 1 bis 3, wobei die Ultraschallbehandlungsbedingungen fünf Wiederholungen einer 30-Sekunden-Ultraschallbehandlung bei 1 MHz, 2 W/cm², relativer Einschaltedauer (duty ratio) 20 % mit einer 10-Sekunden-Pause einschließen.

5. Verfahren nach einem beliebigen der Ansprüche 1 bis 4, wobei das Peptid, Oligopeptid, Polypeptid oder Protein β-Amyloid (Aβ) ist.

6. Verfahren nach einem beliebigen der Ansprüche 1 bis 5, wobei das Fibril oder Aggregat aus Aβ (1-40) oder Aβ (1-42) besteht.

## Revendications

1. Méthode *in vitro* permettant d'identifier si un médicament candidat est utile dans le traitement de la maladie d'Alzheimer (MA) ou le syndrome de Down comprenant :
➢ l'identification si ledit médicament candidat est capable d'éliminer un peptide, un oligopeptide, un polypeptide ou une protéine d'une fibrille ou d'un agrégat en mesurant, en présence dudit médicament candidat, la concentration du peptide soluble, de l'oligopeptide soluble, du polypeptide soluble ou de la protéine soluble dissous/dissoute à partir de la fibrille ou de l'agrégat dans un solvant ;
➢ où le peptide soluble, l'oligopeptide soluble, le polypeptide soluble ou la protéine soluble est dans un état d'équilibre dans le solvant ;
➢ où l'état d'équilibre est obtenu lorsque le taux de dissolution du peptide soluble, de l'oligopeptide soluble, du polypeptide soluble ou de la protéine soluble à partir de la fibrille ou de l'agrégat du peptide, de l'oligopeptide, du polypeptide ou de la protéine dans un solvant est identique au taux d'agrégation du peptide, de l'oligopeptide, du polypeptide ou de la protéine soluble dans le solvant pour former la fibrille ou l'agrégat insoluble, et qu'elles s'équilibrent ; et où l'état d'équilibre est obtenu sous ultrasonication.

2. Méthode selon la revendication 1, dans laquelle la fibrille ou l'agrégat a été formé(e) *in vitro.*

3. Méthode selon la revendication 1 ou 2, dans laquelle l'ultrasonication n'est pratiquement pas accompagnée de génération de chaleur.

4. Méthode selon l'une quelconque des revendications 1 à 3, dans laquelle les conditions de l'ultrasonication comprennent 5 répétitions d'une ultrasonication de 30 secondes à 1 MHz, 2 W/cm², un rapport cyclique de 20 % avec une pause de 10 secondes.

5. Méthode selon l'une quelconque des revendications 1 à 4, dans laquelle le peptide, l'oligopeptide, le polypeptide ou la protéine est le β-amyloïde (Aβ).

6. Méthode selon l'une quelconque des revendications 1 à 5, dans laquelle la fibrille ou l'agrégat est constitué(e) d'Aβ (1-40) ou d'Aβ (1-42).


### SEQUENCE LISTING

<110> AnGesMG, Inc.
<120> Assay method for identifying drug candidate
<130> T64504PCEP
<140> EP 05728425.9
   <141> 2005-03-30
<150> JP 2004-107746
   <151> 2004-03-31
<160> NUMBER OF SEQ ID NOS: 2
   <170> PatentIn Ver. 2.1
<210> 1
   <211> 40
   <212> Peptide
   <213> Homo Sapiens
   <223> OTHER INFORMATION: AƒÀ(1-40)
<400> SEQUENCE: 1
   DAEFRHDSGY EVHHQKLVFF AEDVGSNKGA IIGLMVGGVV 40
<210> 2
   <211> 42
   <212> Peptide
   <213> Homo Sapiens
   <223> OTHER INFORMATION: AƒÀ(1-42)
<400> SEQUENCE: 2
   DAEFRHDSGY EVHHQKLVFF AEDVGSNKGA IIGLMVGGVV IA 42